**Europäisches Patentamt**

**European Patent Office**

**Office européen des brevets**

(19)

(11) Veröffentlichungsnummer: **0 204 968**
**B1**

(12) **EUROPÄISCHE PATENTSCHRIFT**

(45) Veröffentlichungstag der Patentschrift:
**27.09.89**

(21) Anmeldenummer: **86106282.6**

(22) Anmeldetag: **07.05.86**

(51) Int. Cl.⁴: **A61L 15/06, A61L 15/03**

(54) **Nitro-Pflaster.**

(30) Priorität: **24.05.85 DE 3518707**
**15.06.85 EP 85107404**

(43) Veröffentlichungstag der Anmeldung:
**17.12.86 Patentblatt 86/51**

(45) Bekanntmachung des Hinweises auf die Patenterteilung:
**27.09.89 Patentblatt 89/39**

(84) Benannte Vertragsstaaten:
**AT BE CH DE FR GB IT LI LU NL SE**

(56) Entgegenhaltungen:
**EP-A- 0 033 615**
**EP-A- 0 122 344**
**EP-A- 0 169 364**
**DE-A- 3 119 752**
**DE-A- 3 315 245**
**GB-A- 2 073 588**

(73) Patentinhaber: **Beiersdorf Aktiengesellschaft,
Unnastrasse 48, D-2000 Hamburg 20(DE)**

(72) Erfinder: **Guse, Günter, Dr., Liliencronstrasse 30,
D-2000 Hamburg 73(DE)**
Erfinder: **Böss, Johannes, Dr., Siekreystrasse 11,
D-2000 Hamburg 61(DE)**
Erfinder: **Asmussen, Bodo, Dr., Dorotheenweg 1a,
D-2071 Ammerbek(DE)**
Erfinder: **Borchert, Günter, Schiffebker Weg 59,
D-2000 Hamburg 74(DE)**

## Beschreibung

Die Erfindung betrifft selbstklebende Pflaster mit Glycerintrinitrat oder Isosorbiddinitrat in einer Selbstklebemasse aus gesättigtem Kautschuk und klebrigmachendem Herz zur transdermalen Applikation.

Selbstklebende Pflaster zur transdermalen Applikation von Glycerintrinitrat sind bekannt und auch im Handel erhältlich. Bei diesen bekannten Pflastern kann man zwei Typen von Pflastern unterscheiden sowie einen aus diesen beiden herzuleitenden Mischtyp.

Der eine bekannte Pflastertyp ist gekennzeichnet durch eine Steuermembran, durch welche die Abgabe von Glycerintrinitrat in die Haut dosiert wird. Dazu gibt es eine Vielzahl von Publikationen und Patenten bzw. Patentanmeldungen, etwa DE-A 2 135 533, US-A 3 598 123 und US-A 3 797 494. Diese Pflaster finden ihre anwendungstechnische Grenze gesetzt durch den komplizierten Aufbau aus Steuermembran und darauf vorgesehenem Wirkstoffreservoir.

Der zweite Typ der bekannten Pflaster enthält das Glycerintrinitrat in einer mehr oder weniger klebenden Masse, die direkt auf die Haut aufzubringen ist, also ohne Steuermembran. Auch hierzu gibt es diverse Publikationen und Patentanmeldungen, beispielsweise die EP-A 54 279. Praktische Bedeutung hat dieser Typ nicht erlangen können, insbesondere wegen unzureichender Wirkstoffabgabe über einen längeren Zeitraum und die damit einhergehende unzuverlässige therapeutische Wirksamkeit derartiger Pflaster.

Infolge dieser Unzulänglichkeiten wurde auch bereits ein Mischtyp der beiden vorgenannten Pflaster-Typen beschrieben. So betrifft die DE-A 3 315 272 zum einen ein Glycerintrinitrat-Pflaster vom ersten Typ, worin eine Haftklebeschicht die Aufgabe der durchlässigen Steuermembran übernimmt, hinter der sich ein Reservoir von verschieden konzentrierten Schichten befindet. Diese DE-A beschreibt jedoch auch eine Ausführungsform, bei der auch die Haftklebemasse Glycerintrinitrat enthält, so auch das Beispiel 1. Dabei besteht die Selbstklebemasse aus einem gesättigten Kautschuk, nämlich Polyisobutylen mit einem mittleren Molekulargewicht von 900 000 bis 1,4 Mio. Trotz des Konzentrationsgefälles der verschiedenen Masse-Schichten kann ein solches Pflaster unter Praxisbedingungen nicht überzeugen. Insbesondere fallen die Plasmakonzentrationen an Glycerintrinitrat nach mehr als 12 Stunden auf sub-therapeutische Werte ab.

Zwar ist ein Gemisch aus höhermolekularem und niedermolekularem Polyisobuten sowie Mineralöl als Wirkstoffreservoir für die Herstellung von Pflastern zur transdermalen Applikation von Wirkstoffen aus der DE-A 3 119 752 bekannt. Jedoch handelt es sich dabei nicht um eine Selbstklebemasse. Zudem ist für die Herstellung eines gebrauchsfähigen Pflasters auch hier eine Steuermembran und eine zusätzliche Klebemittelschicht zur Befestigung auf der Haut erforderlich.

Aufgabe der Erfindung war es, hier Abhilfe zu schaffen, insbesondere selbstklebende Pflaster mit Glycerintrinitrat oder Isosorbiddinitrat zu schaffen, die einerseits einfach aufgebaut sind und damit wirtschaftlich hergestellt werden können, die andererseits aber über längere Zeit therapeutisch ausreichende Wirkstoff-Blutspiegel ermöglichen.

Demgemäß betrifft die Erfindung selbstklebende Pflaster mit Glycerintrinitrat oder Isosorbiddinitrat, wie dies in den Patentansprüchen gekennzeichnet ist.

Diese Pflaster ermöglichen durch das günstige Zusammenwirken der höhermolekularen, gerüstbildenden Komponente des gesättigten Kautschuks zusammen mit der niedermolekularen, weichmachenden Komponente des gesättigten Kautschuks zum einen den außerordentlich einfachen Aufbau des erfindungsgemäßen Pflasters und damit eine überaus wirtschaftliche Herstellung desselben, zum anderen jedoch eine überaus günstige Abgabe des Glycerintrinitrats oder Isosorbiddinitrats über einen langen Zeitraum mit dem Ergebnis überlegener Blutspiegel. Dabei übernimmt die niedermolekulare Komponente des gesättigten Kautschuks zugleich weichmachende Aufgaben und fördert die Klebrigkeit der daraus hergestellten Selbstklebemasse. In Anbetracht der komplizierten und aufwendig aufgebauten Pflaster des Standes der Technik liegt darin die überraschende Lösung des Problems in wirtschaftlich überzeugender und therapeutisch hochwirksamer Weise. Damit sind die erfindungsgemäßen Pflaster überaus geeignet zur Prophylaxe der Angina pectoris bzw. einem Einsatz, den schon die bisher handelsüblichen Glycerintrinitrat-Pflaster erfahren.

Den Selbstklebemassen können Zusätze, vorzugsweise in Anteilen bis zur fünffachen Wirkstoffmenge (Gewichtsmenge), beigegeben werden, die die Klebeeigenschaften und/oder die Wirkstoffliberation in gewünschter Weise beeinflussen. Dies können je nach liberationskinetischem Erfordernis und gewünschter Anwendungsdauer des Pflasters polare bzw. hydrophile oder weniger polare und unpolare Zuschlagstoffe sein.

Polare bzw. hydrophile Zuschlagstoffe sind vornehmlich mehrwertige Alkohole wie beispielsweise Glycerin, Propandiol-1,2 oder Polyethylenglykole. Als weniger polare oder unpolare Hilfsstoffe kommen vorzugsweise Triglyceride mittelkettiger Fettsäuren, Fettsäureester einwertiger Alkohole oder Paraffinöle in Betracht.

Vorteilhafte Molekulargewichte, Mengenbereiche, Prozentangaben und andere bevorzugte Ausgestaltungen der erfindungsgemäßen Pflaster sind aus den Unteransprüchen zu ersehen.

Zur Herstellung der erfindungsgemäßen Pflaster kann man sich an sich bekannter Techniken bedienen. So können Kautschuk-Harz-Gemische mit Lösungsmitteln in einem Kneter hergestellt werden, wobei als Harze z.B. gesättigte Kohlenwasserstoffharze verwendet werden können. Die Wirkstoffzubereitung wird den Polymerlösungen vorteilhaft durch einen weiteren Mischprozeß hinzugefügt. Es ist auch möglich, die Wirkstoffzubereitung, das Harz und einen Teil des Lösungsmittels zunächst für sich zu mischen und erst dann mit der vorbereiteten Kautschuk-Lösung zu vereinigen.

Die wirkstoffhaltigen Selbstklebemassen können direkt auf die – gegebenenfalls metallisierte, lackierte und vorgestrichene – Trägerfolie gestrichen werden, je nach Art des gewünschten Trockenverfahrens in einem oder in mehreren Strichen. Zur Erleichterung der Trocknung kann die Masse insbesondere in zwei oder drei Strichen aufgetragen werden. Es ist aber auch möglich, die Schicht oder eine Teilschicht auf einen Hilfsträger zu erzeugen und sie dann auf die Trägerfolie zu kaschieren.

Aus der beschichteten und getrockneten Bahn können dann einzelne Pflaster ausgestanzt, mit einer wiederabziehbaren Schutzfolie eingedeckt und in einem Siegelbeutel verpackt werden.

Im folgenden wird die Erfindung beispielhaft erläutert, ohne damit aber ihren Gegenstand unnötig einschränken zu wollen.

GTN = Glycerintrinitrat, GT = Gewichtsteile

Ein Gemisch von

24,9 GT Polyisobutylen $\overline{M_v}$ = 2 800 000
(im Handel erhältlich als Oppanol® B 150)
12,3 GT Polyisobutylen $\overline{M_v}$ = 40 000
(im Handel erhältlich als Oppanol® B 10)
78,8 GT n-Heptan

wird im Kneter zu einer homogenen, viscosen Lösung verarbeitet.

Eine Suspension aus

51,4 GT Lactose-GTN-Verreibung (10% GTN)
22,6 GT aliphatischem Kohlenwasserstoffharz
(Erweichungspunkt R + K 97°C)
14,4 GT Polyisobutylen $\overline{M_v}$ = 40 000
3,9 GT 5%ige Lösung von GTN in Neutralöl
64,9 GT n-Heptan

wird in einem geeigneten Mischer bereitet.

Polyisobutylen-Lösung und Suspension werden durch Rühren zur streichfertigen Selbstklebemasse vereinigt, die einen GTN-Gehalt von 3,38 Gew.-% hat.

Eine Polyethylenterephthalat-Folie von 0,015 mm Stärke, aluminisiert, außenseitig lackiert und mit einem haftvermittelnden Vorstrich versehen, wird mit einer homogenen Schicht der genannten Selbstklebemasse bestrichen. Nachdem das Lösungsmittel bei Raumtemperatur verdampft ist, verbleibt eine homogene, klebrige, wirkstoffhaltige Selbstklebeschicht mit einem Trockengewicht von etwa 360 g/m².

Aus der getrockneten Bahn werden Pflaster von 16 cm² beliebigen Formats (z.B. rund oder oval) geschnitten oder gestanzt, mit abziehbarer Schutzfolie eingedeckt und in Siegelbeutel verpackt. Die Schutzfolie besteht dabei aus einer Polyethylenterephthalat-Folie von 0,1 mm Stärke, aluminisiert und siliconisiert an der an die Selbstklebemasse anliegenden Seite.

Vergleichsversuch

Die Prüfung der erfindungsgemäßen Nitropflaster auf Bioverfügbarkeit des Wirkstoffes im Vergleich zum Stand der Technik erfolgte an freiwilligen herzgesunden Probanden (erfindungsgemäß n = 6, Stand der Technik n = 5). Die Pflaster wurden ihnen auf den Brustkorb geklebt und verblieben dort für 48 Stunden. Ablösungen oder Hautreizungen wurden nicht beobachtet. Den Probanden wurden Blutproben unmittelbar vor der Applikation und dann in zeitlichen Abständen nach der Applikation der Pflaster entnommen. Die Blutproben wurden in bekannter Weise aufbereitet und der Glycerintrinitrat-Gehalt durch GC-MS-Kopplung bestimmt. Die Tabelle zeigt die gemessenen Blutspiegel nach Applikation eines erfindungsgemäßen Pflasters nach Beispiel 1 mit einer Größe von 16 cm² und mit einem Gehalt von 23 mg GTN. Als Vergleich diente ein im Handel erhältliches, entsprechenden Nitropflastern nach dem Stand der Technik DE-A 3 315 272 Beispiel 1, mit einem Gehalt von 17,5 mg GTN. Auch dieses Pflaster hatte eine Größe von 16 cm².

| Zeit nach Applikation (Stunden) | Plasmakonzentration mit erfindungsgemäßem Pflaster nach Beispiel 1 (pg/ml) | Plasmakonzentration mit Pflaster nach DE-A-3 315 272 (pg/ml) |
|---|---|---|
| 0,25 | 13 | 13 |
| 0,5 | 78 | 41 |
| 1 | 64 | 58 |
| 2 | 69 | 74 |
| 4 | 63 | 52 |
| 6 | 59 | 58 |
| 8 | 88 | 64 |
| 12 | 63 | 63 |
| 24 | 49 | 22 |
| 48 | 29 | – |

Man erkennt insbesondere, daß die erfindungsgemäßen GTN-Pflaster im Bereich von 12 bis 24 Stunden die gewünschten nahezu konstanten Blutspiegel erzeugen und sogar bis hin zu 48 Stunden noch merkliche Plasmakonzentrationen an GTN ergeben. Demgegenüber sind die GTN-Blutspiegel, die nach Applikation von Nitropflastern nach dem Stand der Technik gemessen werden, schon nach 24 Stunden auf einen therapeutisch nicht mehr relevanten Wert abgefallen.

Beispiel 2

ISDN = Isosorbiddinitrat
Ein Gemisch von
21,47 Gew.-% Polyisobuten $\overline{M}_v$ = 2 800 000
10,58 Gew.-% Polyisobuten $\overline{M}_v$ = 40 000
67,95 Gew.-% Benzin, Siedebereich 60–95°C
wird im Kneter zu einer homogenen viscosen Lösung verarbeitet.
Eine Suspension aus
21,43 Gew.-% Lactose-ISDN-Verreibung (40% ISDN)
21,43 Gew.-% aliph. Kohlenwasserstoffharz
(Erweichungspunkt R + K 97°C)
8,57 Gew.-% Polyisobuten $\overline{M}_v$ = 40 000
42,86 Gew.-% Benzin, Siedebereich 100–140°C
5,71 Gew.-% Benzin, Siedebereich 60–95°C
wird in einem geeigneten Mischer bereitet.
232 g der Polyisobuten-Lösung, 233,3 g der Suspension und 200 g Benzin, Siedebereich 60–95°C, werden im Kneter zu einer streichfertigen Selbstklebemasse vereinigt.
Eine aluminisierte Polyethylenterephthalat-Folie von 0,025 mm Stärke wird mit einer homogenen Schicht dieser Selbstklebemasse bestrichen. Nach Abdunsten des Lösungsmittels bei Raumtemperatur verbleibt eine Selbstklebeschicht von 280 g/m², deren ISDN-Gehalt bei 10 Gew.-% liegt.
Aus der getrockneten Bahn werden Pflaster von 16 cm² Fläche gestanzt, mit abziehbarer Schutzfolie bedeckt und in Siegelbeutel verpackt.
Ein derartiges 16-cm²-Pflaster liberiert unter sink-Bedingungen 2,41 mg ISDN binnen 23 h in isotonische Kochsalzlösung.

Beispiel 3

In einem Kneter wird ein Gemisch von
24,9 GT Polyisobutylen $\overline{M}_v$ = 2 800 000
26,3 GT Polyisobutylen $\overline{M}_v$ = 40 000
22,6 GT aliph. Kohlenwasserstoffharz
(Erweichungspunkt R + K 97°C)
bereitet.
1,0 GT Moxonidin und 0,6 GT Polyethylenglycol 400 werden in einem Gemisch von 65 GT 1,1,1-Trichlorethan und 35 GT Ethanol bei 40°C klar gelöst.
Die beiden obengenannten Komponenten werden zu einer streichfähigen Selbstklebemasse vereinigt. Mit Hilfe eines geeigneten Drahtrakels wird je eine Schicht der wirkstoffhaltigen Selbstklebemasse auf zwei Polyethylenterephthalat-Folien erzeugt (eine 15 μm starke Folie als Träger und eine siliconisierte 100 μm starke Folie als wiederentfernbare Schutzabdeckung). Beide werden 24 Stunden bei Raumtemperatur getrocknet und dann zusammenkaschiert.

Aus der getrockneten und mit der Siliconfolie bedeckten Bahn werden durch Schneiden oder Stanzen Pflaster beliebiger Größe hergestellt. Sie werden einzeln in ein diffusionsdichtes Primärpackmittel eingeschweißt, beispielsweise einen Flachbeutel aus Polyethylen/Aluminium/Papier-Verbundmaterial.

**Patentansprüche**

1. Selbstklebende Pflaster mit Glycerintrinitrat oder Isosorbiddinitrat in einer Selbstklebemasse aus gesättigtem Kautschuk und klebrigmachendem Harz zur transdermalen Applikation, dadurch gekennzeichnet, daß der gesättigte Kautschuk eine höhermolekulare, gerüstbildende Komponente mit einem mittleren Molekulargewicht von 2 400 000–3 200 000, insbes. 2 800 000 von 85–40 Gew.-%, insbesondere von 55–45 Gew.-% und eine niedermolekulare, weichmachende Komponente mit einem mittleren Molekulargewicht von 30 000–50 000 insbes. 40 000 von 15–60 Gew.-%, insbes. von 45–55 Gew.-% aufweist.

2. Pflaster nach Anspruch 1, dadurch gekennzeichnet, daß der gesättigte Kautschuk Polyisobutylen ist.

3. Pflaster nach einem der Ansprüche 1 oder 2, dadurch gekennzeichnet, daß das klebrigmachende Harz ein aliphatisches Kohlenwasserstoffharz ist.

4. Pflaster nach einem der Ansprüche 1 bis 3, dadurch gekennzeichnet, daß die Selbstklebemasse von 40–70, insbes. von 45–55 Gew.-% gesättigtem Kautschuk, von 60–30, insbes. von 55–45 Gew.-% klebrigmachendes Harz enthält.

5. Pflaster nach einem der Ansprüche 1 bis 4, dadurch gekennzeichnet, daß das Glycerintrinitrat zusammen mit einem Phlegmatisierungsmittel, insbes. mit 90–95 Gew.-% (bezogen auf die Summe beider) Lactose, vorliegt.

6. Pflaster nach einem der Ansprüche 1 bis 4, dadurch gekennzeichnet, daß das Isosorbiddinitrat zusammen mit einem Phlegmatisierungsmittel, insbes. mit 60% Lactose (bezogen auf die Summe beider) vorliegt.

7. Pflaster nach einem der Ansprüche 1 bis 5, dadurch gekennzeichnet, daß die Selbstklebemasse enthaltend Glycerintrinitrat in einer Menge von 100–500 g/m², insbes. von 200–400 g/m² aufgetragen ist.

8. Pflaster nach einem der Ansprüche 1 bis 6, dadurch gekennzeichnet, daß ein 16 cm² großes Einzel-Pflaster 20–40 mg Glycerintrinitrat enthält.

9. Pflaster nach einem der Ansprüche 1 bis 7, dadurch gekennzeichnet, daß der Träger und/oder eine abziehbare Schutzfolie aus einer undurchlässigen Folie besteht, insbes. aus einer metallisierten Kunststoff-Folie.

10. Verwendung einer Selbstklebemasse aus gesättigtem Kautschuk und klebrigmachendem Harz zur transdermalen Wirkstoff-Applikation von Glycerintrinitrat oder Isosorbiddinitrat, dadurch gekennzeichnet, daß diese Selbstklebemasse eine Zusammensetzung gemäß einem der Ansprüche 1 bis 4 aufweist.

**Revendications**

1. Emplâtre autocollant contenant du trinitrate de glycérine ou du dinitrate d'isosorbide dans une masse autocollante formée de caoutchouc saturé et de résine qui rend adhésif pour l'administration transdermique, caractérisé en ce que le caoutchouc saturé comprend un composant de haut poids moléculaire structurant d'un poids moléculaire moyen de 2 400 000–3 200 000 et en particulier de 2 800 000 pour 85–40% en poids et en particulier pour 55–45% en poids et un composant de bas poids moléculaire plastifiant d'un poids moléculaire moyen de 30 000–50 000 et en particulier de 40 000 pour 15–60% en poids et en particulier pour 45–55% en poids.

2. Emplâtre suivant la revendication 1, caractérisé en ce que le caoutchouc saturé est du polyisobutylène.

3. Emplâtre suivant l'une quelconque des revendications 1 et 2, caractérisé en ce que la résine qui rend adhésif est une résine d'hydrocarbure aliphatique.

4. Emplâtre suivant l'une quelconque des revendications 1 à 3, caractérisé en ce que la masse autocollante contient 40–70 et en particulier 45–55% en poids de caoutchouc saturé et 60–30 et en particulier 55–45% en poids de résine qui rend adhésif.

5. Emplâtre suivant l'une quelconque des revendications 1 à 4, caractérisé en ce que le trinitrate de glycérine est présent conjointement avec un agent de flegmatisation, en particulier avec 90–95% en poids (sur la base de la somme des deux) de lactose.

6. Emplâtre suivant l'une quelconque des revendications 1 à 4, caractérisé en ce que le dinitrate d'isosorbide est présent conjointement avec un agent de flegmatisation, en particulier avec 60% de lactose (sur la base de la somme des deux).

7. Emplâtre suivant l'une quelconque des revendications 1 à 5, caractérisé en ce que la masse autocollante contenant du trinitrate de glycérine est appliquée en une quantité de 100–500 g/m² et en particulier de 200–400 g/m².

EP 0 204 968 B1

8. Emplâtre suivant l'une quelconque des revendications 1 à 6, caractérisé en ce qu'un emplâtre unitaire d'une surface de 16 cm² contient 20–40 mg de trinitrate de glycérine.

9. Emplâtre suivant l'une quelconque des revendications 1 à 7, caractérisé en ce que le support et/ou une feuille de protection amovible consiste en une feuille imperméable, en particulier en une feuille de matière palstique métallisée.

10. Utilisation d'une masse autocollante formée de caoutchouc saturé et de résine qui rend adhésif pour l'administration transdermique de trinitrate de glycérine ou de dinitrate d'isosorbide comme prinicipe actif, caractérisée en ce que la masse auto-collante a une composition suivant l'une quelconque des revendications 1 à 4.

**Claims**

1. Self-adhesive plasters with glycerol trinitrate or isosorbide dinitrate in a self-adhesive composition of a saturated rubber and a tackifying resin for transdermal administration, characterized in that the saturated rubber comprises 85–40% by weight, in particular 55–45% by weight of a higher-molecular, structure-forming component having a mean molecular weight of 2,400,000–3,200,000, in particular 2,800,000, and 15–60% by weight, in particular 45–55% by weight, of a low-molecular, plasticizing component having a mean molecular weight of 30,000–50,000, in particular 40,000.

2. Plaster according to Claim 1, characterized in that the saturated rubber is polyisobutylene.

3. Plaster according to one of Claims 1 or 2, characterized in that the tackifying resin is an aliphatic hydrocarbon resin.

4. Plaster according to one of Claims 1 to 3, characterized in that the self-adhesive composition contains 40–70, in particular 45–55% by weight of saturated rubber and 60–30, in particular 55–45% by weight of tackifying resin.

5. Plaster according to one of Claims 1 to 4, characterized in that the glycerol trinitrate is present together with a stabilizer, in particular with 90–95% by weight of lactose (relative to the sum of the two).

6. Plaster according to one of Claims 1 to 4, characterized in that the isosorbide dinitrate is present together with a stabilizer, in particular with 60% of lactose (relative to the sum of the two).

7. Plaster according to one of Claims 1 to 5, characterized in that the self-adhesive composition containing glycerol trinitrate has been applied in a quantity of 100–500 g/m², in particular 200–400 g/m².

8. Plaster according to one of Claims 1 to 6, characterized in that an individual plaster of 16 cm² size contains 20–40 mg of glycerol trinitrate.

9. Plaster according to one of Claims 1 to 7, characterized in that the carrier and/or a peel-off protective film consist of an impermeable film, in particular a metallized plastic film.

10. Use fo a self-adhesife composition of a saturated rubber and a tackifying resin for transdermal administration of glycerol trinitrate or isosorbide dinitrate as an active compound, characterized in that this self-adhesive composition is composed in accordance with one of Claims 1 to 4.